# EUROPÄISCHE PATENTSCHRIFT

(11) **EP 1 687 301 B1**
(45) Veröffentlichungstag und Bekanntmachung des Hinweises auf die Patenterteilung: **13.05.2009**
(21) Anmeldenummer: 04798099.0
(22) Anmeldetag: 26.11.2004
(51) Int. Cl.: C07D 417/06, C07D 411/06, C07C 259/00, A61K 31/41, A61K 31/435

(54) **THIAEPOTHILONE ZUR BEHANDLUNG VON KREBSERKRANKUNGEN**
THIAEPOTHILONE FOR TREATING CANCEROUS DISEASES
THIAEPOTHILONE POUR TRAITER DES MALADIES CANCEREUSES

(30) Priorität: 26.11.2003 DE 10355223
(43) Veröffentlichungstag der Anmeldung: 09.08.2006
(73) Patentinhaber: LEIBNIZ-INSTITUT FÜR PFLANZENBIOCHEMIE (IPB), 06120 HALLE (SAALE) (DE)
(72) Erfinder: WESSJOHANN, Ludger A., 06120 Halle (Saale) (DE); EICHELBERGER, Uwe, 04509 Priester (DE); TRAN THI PHOUNG, Thao, Dong Da Ha Noi (VN)
(74) Vertreter: Maiwald Patentanwalts GmbH
(86) Internationale Anmeldenummer: PCT/EP2004/013451
(87) Internationale Veröffentlichungsnummer: WO 2005/051947

(56) Entgegenhaltungen:
- WO-A-03/078411
- DE-A1- 4 138 042
- DE-A1- 19 820 599
- TERASAWA ET AL.: "Reaction of Mixed Carboxylic Anhydrides with Grignard Reagents" TETRAHEDRON, Bd. 33, 1977, Seiten 595-598, XP002318906

## Beschreibung

Epothilone (DE 4138042) sind Naturstoffe mit außerordentlicher biologischer Wirkung, z.B. als Mitosehemmer, Mikrotubuli-modifizierende Agenzien, Cytotoxica oder Fungizide. Insbesondere verfügen sie über Paclitaxel-ähnliche Eigenschaften und übertreffen Paclitaxel (Taxol^{®}) in einigen Tests noch an Aktivität. Einige Derivate befinden sich derzeit in klinischen Studien zur Behandlung von Krebsleiden (Nicolaou et al. Angew. Chem. Int. Ed. 1998, 37, 2014-2045; Flörsheimer et al. Expert Opin. Ther. Patents 2001, 11, 951-968).

Die Internationale Patentanmeldung WO 03/078411 betrifft 3-Cyano substituierte Epothilon-Derivate, die antiproliferative Eigenschaften aufweisen, sowie ferner Syntheseverfahren zu deren Herstellung und deren Verwendung bei der Behandlung von Tumorerkrankungen.

Die Deutsche Patentanmeldung DE 198 20 599 A1 offenbart ebenfalls verschiedene, an der C3-Position modifizierte Epothilon-Derivate sowie deren Synthese bzw. Verwendung zur Herstellung von Arzneimitteln und Pflanzenschutzmitteln.

Terasawa und Okada (Tetrahedron (1999) 33, 595-598) beschreiben die Umsetzung von Carboxylsäure-anhydriden mit Grignard Reagenzien im Rahmen der Herstellung von Keto-estern, wobei (2-Oxo-butylsulfanyl)-essigsäure als Synthesezwischenprodukt auftritt.

Ziel der vorliegenden Erfindung war es, neue epothilonartige Derivate bereitzustellen, die ein besseres Profil bezüglich ihres präklinischen und klinischen Entwicklungspotentials aufweisen.

Die vorliegende Erfindung betrifft Verbindungen der allgemeinen Formel (I): 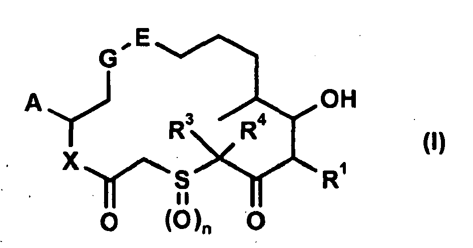 worin
A ein Heteroalkyl-, ein Heterocycloalkyl-, ein Heteroalkylcycloalkyl-, ein Heteroaryl- oder ein Heteroarylalkylrest ist, bevorzugt ein Heteroarylalkylrest oder ein Heteroarylrest,
G-E aus folgenden Gruppen ausgewählt ist, 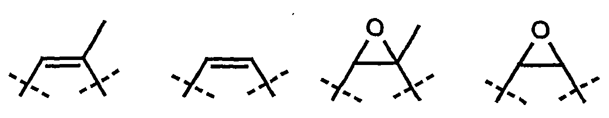 oder Teil eines gegebenenfalls substituierten Cyclopropylrings ist, wobei die bevorzugte Methylgruppe auch durch eine andere Alkygruppe ersetzt sein kann, wobei die Gruppe -(CH)=C(Me)- bevorzugt ist,
n gleich 0, 1 oder 2 ist,
R¹ Wasserstoff, eine C₁-C₄-Alkyl- oder eine C₃-C₄-Cycloalkylgruppe ist, bevorzugt eine C₁-C₄-Alkyl- oder eine C₃-C₄-Cycloalkylgruppe, insbesondere bevorzugt eine Methylgruppe,
X ein Sauerstoffatom oder eine Gruppe der Formel NR² ist, wobei R² ein Wasserstoffatom, OH, NH₂, NH(Alkyl), N(Alkyl)₂, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocyclo-alkyl-, Aralkyl- oder ein Heteroaralkylrest ist und
R³ und R⁴ unabhängig voneinander ein Wasserstoffatom, eine C₁-C₄-Alkylgruppe oder zusammen Teil einer Cycloalkylgruppe mit 3 oder 4 Ringatomen sind, bevorzugt C₁-C₄-Alkylgruppe, besonders bevorzugt Methyl,
oder ein pharmakologisch akzeptables Salz, Solvat, Hydrat oder eine pharmakologisch akzeptable Formulierung derselben.

Der Ausdruck Alkyl bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1 bis 6 (d.h. 1, 2, 3, 4, 5 oder 6) Kohlenstoffatome aufweist, z.B. die Methyl-, Ethyl-, Isopropyl-, Isobutyl-, tert-Butyl, n-Hexyl-, 2,2-Dimethylbutyl- oder n-Octylgruppe.

Die Ausdrücke Alkenyl und Alkinyl beziehen sich auf zumindest teilweise ungesättigte, geradkettige oder verzweigte Kohlenwasserstoffgruppen, die 2 bis 20 Kohlenstoffatome, vorzugsweise 2 bis 12 Kohlenstoffatome, besonders bevorzugt 2 bis 6 (d.h. 2, 3, 4, 5 oder 6) Kohlenstoffatome aufweisen, z. B. die Allyl-, Acetylenyl-, Propargyl-, Isoprenyl- oder Hex-2-enyl-Gruppe.

Der Ausdruck Heteroalkyl bezieht sich auf eine Alkyl-, eine Alkenyl- oder eine Alkinyl-Gruppe, wie vorstehend definiert, in der ein oder mehrere (bevorzugt 1, 2 oder 3) Kohlenstoffatome durch ein Sauerstoff-, Stickstoff-, Phosphor-, Bor- oder Schwefelatom ersetzt sind (bevorzugt Sauerstoff, Schwefel oder Stickstoff), z.B. eine Alkyloxy-Gruppe wie z.B. Methoxy oder Ethoxy, oder eine Methoxymethyl-, Nitril-, Methylcarboxyalkylester- oder 2,3-Dioxyethyl-Gruppe. Der Ausdruck Heteroalkyl bezieht sich des weiteren auf eine Carbonsäure oder eine von einer Carbonsäure abgeleitete Gruppe wie z. B. Acyl, Acyloxy, Carboxyalkyl, Carboxyalkylester z.B. Methylcarboxyalkylester, Carboxy-alkylamid, Alkoxycarbonyl oder Alkoxycarbonyloxy.

Der Ausdruck Cycloalkyl bzw. Cyclo- bezieht sich auf eine gesättigte oder teilweise ungesättigte cyclische Gruppe, die einen oder mehrere Ringe aufweist, bevorzugt 1 oder 2 Ringe), die ein Gerüst bilden, welches 3 bis 14 Kohlenstoffatome, vorzugsweise 3 bis 10 Kohlenstoffatome enthält, z.B. die Cyclopropyl-, Cyclohexyl-, Tetralin- oder Cyclohex-2-enyl-Gruppe.

Der Ausdruck Heterocycloalkyl bzw. Heterocyclo- bezieht sich auf eine Cycloalkylgruppe wie oben definiert, in der ein oder mehrere (bevorzugt 1, 2 oder 3) Kohlenstoffatome durch ein Sauerstoff-, Stickstoff-, Phosphor- oder Schwefelatom ersetzt sind (bevorzugt Sauerstoff, Schwefel oder Stickstoff) und kann beispielsweise für die Piperidin-, Morpholin-, Tetrahydrofuran-, Tetrahydrothiophen-, N-Methylpiperazin- oder N-Phenylpiperazin-Gruppe stehen.

Die Ausdrücke Alkylcycloalkyl bzw. Heteroalkylcycloalkyl beziehen sich auf Gruppen, die entsprechend den obigen Definitonen sowohl Cycloalkyl- bzw. Heterocycloalkyl- wie auch Alkyl-, Alkenyl-, Alkinyl- und/oder Heteroalkylgruppen enthalten.

Der Ausdruck Aryl bzw. Ar bezieht sich auf eine aromatische Gruppe, die einen oder mehrere Ringe hat, bevorzugt 1 oder 2, und durch ein Gerüst gebildet wird, das 5 bis 14 Kohlenstoffatome, vorzugsweise 5 oder 6 bis 10 Kohlenstoffatome enthält z.B. eine Phenyl-, Naphthyl-, 2-, 3- oder 4-Methoxyphenyl-, 2-, 3- oder 4-Ethoxyphenyl-, 4-Carboxyphenylalkyl- oder 4-Hydroxyphenyl-Gruppe.

Der Ausdruck Heteroaryl bezieht sich auf eine ArylGruppe, in der ein oder mehrere (bevorzugt 1, 2 oder 3) Kohlenstoffatome durch ein Sauerstoff-, Stickstoff-, Phosphor- oder Schwefelatom (bevorzugt Sauerstoff, Schwefel oder Stickstoff) ersetzt sind, z.B. die 4-Pyridyl-, 2-Imidazolyl-, 3-Pyrazolyl-, Oxazolyl-, Thiazolyl-, Thiophen- und Isochinolinyl-Gruppe.

Die Ausdrücke Aralkyl bzw. Heteroaralkyl beziehen sich auf Gruppen, die entsprechend den obigen. Definitionen sowohl Aryl- bzw. Heteroaryl- wie auch Alkyl-, -Alkenyl-, Alkinyl- und/oder Heteroalkyl- und/oder Cycloalkyl- und/oder Heterocycloalkylgruppen enthalten, z.B. die Tetrahydroisochinolinyl-, Benzyl-, 2- oder 3-Ethylindolyl- oder 4-Methylpyridino-Gruppe.

Die Ausdrücke Alkyl, Alkenyl, Alkinyl, Heteroalkyl, Cycloalkyl, Heterocycloalkyl, Aryl, Heteroaryl, Aralkyl und Heteroaralkyl umfassen die nichtsubstituierten Gruppen aber auch die jeweiligen Gruppen in substituierter Form. Diese substituierten Gruppen, ggf. gekennzeichnet durch den Ausdruck "gegebenenfalls substituiert" beziehen sich auf Gruppen, in denen ein oder mehrere Wasserstoffatome (bevorzugt 1, 2 oder 3, insbesondere 1 oder 2) solcher Gruppen durch Fluor-, Chlor-, Brom- oder Jodatome oder OH, SH, NH, oder NO₂-Gruppen ersetzt sind. Diese Ausdrücke beziehen sich weiterhin auf Gruppen, die mit unsubstituierten Alkyl-, Alkenyl-, Alkinyl-, Heteroalkyl-, Cycloalkyl-, Heterocycloalkyl-, Aryl-, Heteroaryl-, Aralkyl- oder Heteroaralkyl-Gruppen substituiert sind (bevorzugt sind Alkyl-, Alkenyl-, Alkinyl-, und Arylgruppen) (d.h. es sind ein oder mehrere Wasserstoffatome (bevorzugt 1, 2 oder 3, insbesondere 1 oder 2) durch diese Gruppen ersetzt), wobei diese wie vorstehend definiert sind.

Verbindungen der Formel (I) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (I) sowie Gemische davon umfasst.

Bevorzugt sind Verbindungen der Formel (I) wobei A eine Gruppe der Formel -C(CH₃)=CHR⁵, -C(C₂H₅)=CHR⁵, -C(C1)=CHR⁵ oder -CH=CHR⁵ ist, wobei R⁵ ein Heteroaryl- oder ein Heteroarylalkylrest ist.

Des weiteren bevorzugt sind Verbindungen der Formel (I) wobei A die allgemeine Formel (II) bis (V), bevorzugt (II) oder (III) aufweist: 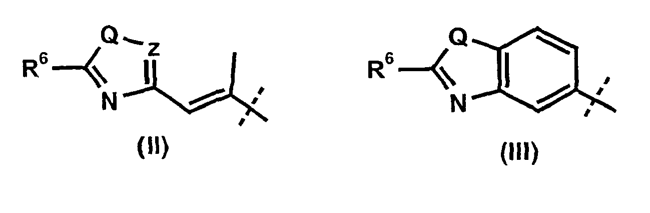 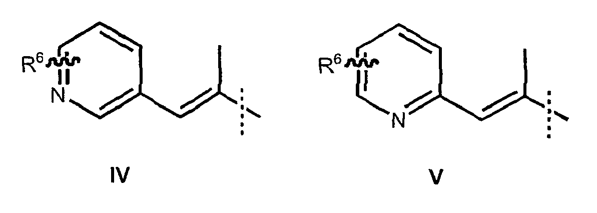 wobei Q ein Schwefelatom, ein Sauerstoffatom oder eine Gruppe der Formel NR⁷ ist, wobei R⁷ ein Wasserstoffatom, eine C₁-C₄-Alkylgruppe oder eine C₁-C₄-Heteroalkylgruppe ist, z ein Stickstoffatom oder eine CH-Gruppe ist und R⁶ eine Gruppe der Formel OR⁸ oder NHR⁸, eine Alkyl-, Alkenyl, Alkinyl- oder eine Heteroalkylgruppe (bevorzugt eine Gruppe der Formel CH₂OR⁸ oder CH₂NHR⁸) ist, wobei R⁸ ein Wasserstoffatom, eine C₁-C₄-Alkylgruppe oder eine C₁-C₄-Heteroalkylgruppe (bevorzugt ein Wasserstoffatom) ist. Bevorzugt sind die Gruppen (II), (III) sowie, (V), insbesondere die Gruppe (II). R⁶ ist bevorzugt eine Alkylgruppe, insbesondere Methyl.

Besonders bevorzugt ist z eine CH-Gruppe.

Wiederum bevorzugt sind Verbindungen der Formel (I) wobei Q ein Schwefelatom oder ein Sauerstoffatom ist, insbesondere ein schwefelatom, bevorzugt in der Gruppe (II).

Besonders bevorzugt sind Verbindungen der Formel (I) wobei R⁶ eine Gruppe der Formel CH₃, CH₂OH oder CH₂NH₂ ist.

Weiter bevorzugt ist R² ein Wasserstoffatom oder eine C₁-C₄-Alkylgruppe (besonders bevorzugt ein Wasserstoffatom).

Des weiteren bevorzugt sind Verbindungen der Formel (I) wobei X ein Sauerstoffatom ist.

Ausserdem ist R¹ bevorzugt eine Methyl, Ethyl oder eine Propylgruppe; besonders bevorzugt eine Methylgruppe.

Wiederum bevorzugt sind R³ und R⁴ Methylgruppen.

Beispiele für pharmakologisch akzeptable Salze der Verbindungen der Formel (I) sind Salze (oder Mischsalze) von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Maleinsäure und Salicylsäure. Verbindungen der Formel (I) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (I) auftreten. Wenn die Verbindungen der Formel (I) asymmetrische C-Atome enthalten, können sie entweder als achirale Verbindungen, Diastereomeren-Gemische, Gemische von Enantiomeren oder als optisch reine Verbindungen vorliegen. Des weiteren sind von der vorliegenden Erfindung auch alle cis/trans-Isomeren der vorliegenden Verbindungen der allgemeinen Formel (I) sowie Gemische davon umfasst.

Die pharmazeutischen Zusammensetzungen gemäß der vorliegenden Erfindung enthalten mindestens eine Verbindung der Formel (I) als Wirkstoff und fakultativ Trägerstoffe und/oder Adjuvantien.

Die Pro-Drugs (siehe z. B. R. B. Silverman, Medizinische Chemie, VCH Weinheim, 1995, Kapitel 8, S. 361ff), die ebenfalls Gegenstand der vorliegenden Erfindung sind, bestehen aus einer Verbindung der Formel (I) und mindestens einer pharmakologisch akzeptablen Schutzgruppe, die unter physiologischen Bedingungen abgespalten wird, z.B. einer Alkoxy-, Aralkyloxy-, Acyl- oder Acyloxy-Gruppe, wie z.B. einer Ethoxy-, Benzyloxy-, Acetyl- oder Acetyloxy-Gruppe.

Die therapeutische Verwendung der Verbindungen der Formel (I), ihrer pharmakologisch akzeptablen Salze bzw. Solvate und Hydrate sowie Formulierungen und pharmazeutischen Zusammensetzungen liegt ebenfalls im Rahmen der vorliegenden Erfindung.

Auch die Verwendung dieser Wirkstoffe zur Herstellung von Arzneimitteln zur Behandlung von Krebserkrankungen ist Gegenstand der vorliegenden Erfindung. Im allgemeinen werden Verbindungen der Formel (I) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff, Edelgase und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können weitere Wirkstoffe beinhalten, die gewöhnlich zur Behandlung von Krebserkrankungen eingesetzt werden.

Zur Behandlung von Krebserkrankungen kann die Dosis der erfindungsgemäßen biologisch aktiven Verbindung innerhalb breiter Grenzen variieren und kann auf den individuellen Bedarf eingestellt werden. Im allgemeinen ist eine Dosis von 1 µg bis 100 mg/kg Körpergewicht pro Tag geeignet, wobei eine bevorzugte Dosis 10 µg bis 25 mg/kg pro Tag ist. In geeigneten Fällen kann die Dosis auch unter oder über den oben angegebenen Werten liegen.

### Beispiele

Die Synthese des "northern half alcohol" ist in WO0232844 beschrieben

### Baustein A (Südhälfte)

### A1)

### 2-Brom-2-methyl-pentan-3-on

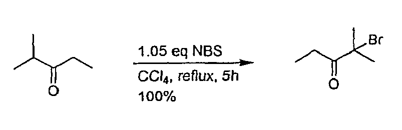

2-Methyl-3-pentanon (10g, 100 mmol) wird in CCl₄ (50 ml) gelöst und mit NBS (18.7 g, 105 mmol) und einer katalytische Menge AIBN versetzt. Die Mischung wird 5 h bei rt unter Bestrahlung mit einer 100 W Lichtquelle gerührt. Der entstandene Feststoff wird abfiltriert und das Filtrat mit Wasser (2 x 20 ml), NaHCO₃ (1 x 20 ml) und wiederum mit Wasser (2 x 20 ml) gewaschen. Trocknen über Na₂SO₄ und Abziehen des Lösungsmittels im Vakuum ergeben das Rohprodukt als gelbes Öl, welches durch Säulenchromatographie (PE : EE 25:1) gereinigt wird.
¹H-NMR (400 MHz, CDCl₃): δ = 1.13 (t, *J*=7.4 Hz, 3H), 1.87 (s, 6H), 2.85 (q, *J*=7.4 Hz, 2H)

### A2)

### (1,1-Dimethyl-2-oxo-butylsulfanyl)-essigsäureethylester

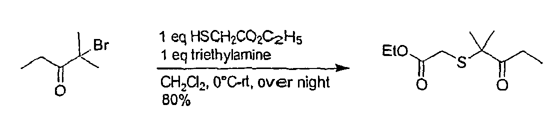

Zu einer Lösung von 2-Brom-2-methyl-pentan-3-on (A1) (13.5g, 75 mmol) in CH₂Cl₂ (400 ml) und NEt₃ (7.57 g,

10.42 ml, 75 mmol) wird unter Rühren bei 0°C langsam eine Lösung von 2-Mercaptoessigsäureethylester (9 g, 8.2 ml, 75 mmol) in CH₂Cl₂ (40 ml) zugetropft. Es wird 3 h bei 0°C und weitere 12 h bei RT gerührt. Die Reaktionsmischung wird mit Wasser (100 ml) und Lake (brine) (100 ml) gewaschen, über Na₂SO₄ getrocknet und das Lösungsmittel im Vakuum abgezogen. Das Rohprodukt wird mittels Säulenchromatographie (PE : EE 25:1) gereinigt.
¹H-NMR (400 MHz, CDCl₃) δ = 1.06 (t, *J*=7.0 Hz, 3H), 1.15 (t, *J*=7.0 Hz, 3H), 1.44 (s, 6H), 2.74 (q, *J*=7.4 Hz, 2H), 3.15 (s, 2H), 4.15 (q, *J*=7.42 Hz, 2H),

### A3)

### (1,1-Dimethyl-2-oxo-butylsulfanyl)-essigsäure

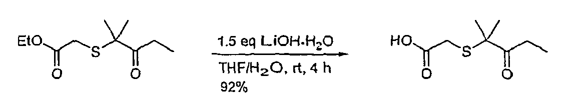

Zu einer Lösung von (1,1-Dimethyl-2-oxo-butylsulfanyl)-essigsäureethylester (A2) (18 g, 82 mmol) in einem THF/H₂O-Gemisch (400 ml 3:1) wird LiOH (6.88 g, 164 mmol) gegeben und die Mischung für 4 h bei rt gerührt. Es wird mit Wasser (100 ml) verdünnt und die Mischung mit HCl auf pH 2 angesäuert. Die wässrige Phase wird mit Et₂O (2 x 100 ml) extrahiert und die vereinigten org. Phasen mit Wasser (100 ml) gewaschen, über Na₂SO₄ getrocknet und im Vakuum eingeengt. Das Rohprodukt wird mittels Säulenchromatographie (PE : EE 1:1 + 1% Essigsäure) gereinigt.
¹H-NMR (400 MHz, CDCl₃) : δ =1.05 (t, *J*=7.0 Hz, 3H), 1.25 (s, 1H), 1.47 (s, 6H), 2.73 (q, *J*=7. 0 Hz, 2H), 3.21 (s, 2H)

### A4)

### (2-Methyl-3-oxo-pentan-2-sulfinyl)-essigsäure (TPT 173)

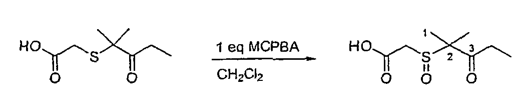

Zu einer Lösung von (1,1-Dimethyl-2-oxo-butylsulfanyl)-essigsäure (A3) (0.25 g, 1.32 mmol) in CH₂Cl₂ (5 ml) wird Metachlorperbenzoesäure (0.23 g, 1.32 mmol) gegeben und die Mischung bei 0°C für 3 h gerührt. Es wird mit Wasser (5 ml) verdünnt, mit Essigsäure angesäuert und mit Ethylacetat (3 x 10 ml) extrahiert. Die organische Phase wird über Na₂SO₄ getrocknet und das Lösungsmittel im Vakuum abgezogen. Das Rohprodukt wird mittels Säulenchromatographie (PE : EE 1:1 + 1% Essigsäure) gereinigt.
¹H-NMR (300 MHz, CDCl₃): δ = 0.98-1.05 (m, 3H, CH₃-5), 1.43, 1.48 (2 x s, 6H, CH₃-1, 2-CH₃), 2.53-2.79 (m, 2H, CH₂-4), 3.37, 3.54 (2 x d, 2H, SCH₂), 8.82 (bs, 1H, OH) C₈H₁₄O₄S (206.26, 206.06), HRMS: ber. (M+Na)⁺ 229.0505, gef. 229.0510

### A5)

### (3-Brom-1,1-dimethyl-2-oxo-butylsulfanyl)-essigsäure (DUE 214)

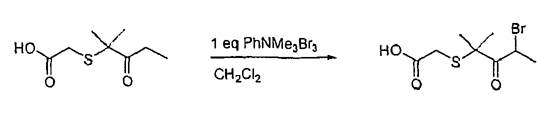

Zu einer Lösung von (1,1-Dimethyl-2-oxo-butylsulfanyl)-essigsäure (A3) (0.3 g, 1.58 mmol) in CH₂Cl₂ (10 ml) wird PhNMe₃⁺Br⁻ (0.62 g, 1.66 mmol) gegeben und die Mischung für 20 min bei 0°C und weitere 60 min bei RT gerührt. Es wird Wasser (20 ml) zugefügt, mit Et₂O (3 x 30 ml) extrahiert und die organische Phase mit HCl (1N, 30 ml) und brine (30 ml) gewaschen. Trocknen über Na₂SO₄ und Entfernen des Lösungsmittels im Vakuum ergeben das Rohprodukt, welches durch Säulenchromatographie (PE : EE 1:1 + 1% Essigsäure) gereinigt wird.
¹H-NMR (300 MHz, CDCl₃): δ = 1.15, 1.64 (2 x s, 6H, 1-(CH₃)₂), 1.81 (d, 3H, CH₃-4), 3.18 (dd, 2H, SCH₂), 5.07 (q, 1H, CH-3)
C₈H₁₃BrO₃S (269.16, 267.98), HRMS: ber. (M+Na)⁺ 290.9661, gef. 290.9669

### A6)

### (4-Brom-2-methyl-3-oxo-pentan-2-sulfonyl)-essigsäure (TPT141_1)

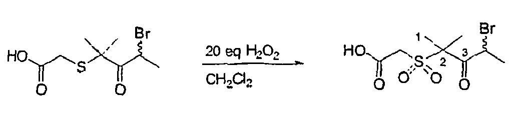

Zu einer Lösung von (3-Brom-1,1-dimethyl-2-oxobutylsulfanyl)-essigsäure (A5) (0.77 g, 2.9 mmol) in Essigsäure (6 ml) wird 30 % H₂O₂ (1.91 g, 5.74 ml, 56 mmol) zugegeben und die Mischung 4 h bei RT gerührt. Es wird mit Ethylacetat (3 x 10 ml) extrahiert, über Na₂SO₄ getrocknet und das Lösungsmittel im Vakuum abgezogen. Das Rohprodukt wird mittels Säulenchromatographie (PE : EE 1:1 + 1% Essigsäure) gereinigt.
¹H-NMR (300 MHz, CDCl₃): δ = 1.77, 1.97 (2 x s, 6H, CH₃-1, 2-CH₃), 1.80 (d, 3H, CH₃-5), 4.03, 4.20 (2 x d, 2H, SO₂CH₂), 4.74 (bs, 1H, OH), 4.92 (q, 1H, CH-4) C₈H₁₃BrO₅S (301.15, 299.97), ESI-MS: (M-H) 299.03

### A7)

### (2-Oxo-propylsulfanyl)-essigsäureethylester

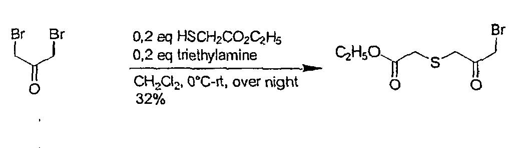

Entsprechend Vorschrift A2 wird ein Überschuß Dibromaceton (sehr tränenreizend) umgesetzt. Die resultierende Verbindung ist sehr hydrolyseempfindlich und sollte möglichst wasserfrei und bei tiefer Temperatur aufbewahrt werden.
C₇H₁₁BrO₃S (254) - gef. 255 (M+H)

### Baustein B (Nordhälfte)

### B1)

### tert-Butyl 2-acetoxyacetoacetat

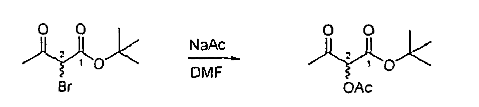

Zu einer Suspension von Natriumacetat (30.76 g, 375 mmol) in DMF (250 mL) wird tropfenweise *tert*-Butyl 2-bromoacetoacetat (59.27 g, 250 mmol) gegeben. Nach 90 minütigem Rühren bei RT wird Wasser (415 ml) zugefügt und mit Ethylacetat (3 x 325 ml) extrahiert. Die vereinigten organischen Extrakte werden mit Wasser (3 x 325 ml) und brine (325 ml) gewaschen, über Na₂SO₄ getrocknet und das Lösungsmittel im Vakuum abgezogen. Das resultierende Öl wird durch Destillation (12 mbar, 128°) gereinigt.
¹H-NMR (200 MHz, CDCl₃): δ = 1.50 (s, 9H, C(CH₃)₃), 2.22 (s, 3H, CH₃COO), 2.34 (s, 3H, CH₃CO), 5.41 (s, 1H, CH) ppm C₁₀H₁₆O₅ (216.23, 216.10), MS (CI): m/z (%) = 117 (19), 143 (12), 161 (100), 205 (43), 207 (12), 217 (18); HRMS: ber. (MH⁺) 217.1076, gef. 217.1046.

### B2)

### Z-2-Acetoxy-2-acetyl-5,9-dimethyl-deca-4,8-dien-säure-tert-butylester

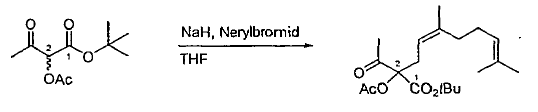

*tert*-Butyl 2-acetoxyacetoacetat (B1) (19.5 g, 90 mmol) wird bei 0°C tropfenweise zu einer gerührten Suspension von NaH (2.59 g, 108 mmol) in THF (180 mL) gegeben. Nach beendeter Gasentwicklung wird Nerylbromid (19.6 g, 90 mmol) ebenfalls tropfenweise bei 0°C zugefügt und die Mischung für weitere 16 h bei RT gerührt. Es wird mit Et₂O (750 mL) verdünnt, mit Wasser (3x200 mL) und brine (1×200 mL) gewaschen, über Na₂SO₄ getrocknet und das Lösungsmittel im Vakuum entfernt. Das Rohprodukt kann ohne weitere Reingungsschritte weiterverwendet werden.
¹H-NMR (400 MHz, CDCl₃): δ = 1.46 (s, 9H, C(CH₃)₃), 1.60 (s, 3H, =C(CH₃)), 1.68 (s, 3H, =C(CH₃)), 1.70 (s, 3H, =C(CH₃)), 2.00-2.05 (m, 4H, CH₂-6, CH₂-7), 2.16 (s, 3H, CH₃COO), 2.31 (s, 3H, CH₃CO), 2.82-2.87 (m, 2H, CH₂-3), 5.00-5.09 (m, 2H, CH-4, CH-8) ppm.
MS (CI): m/z (%) = 353 (13) (MH⁺), 298 (21), 297 (100), 279 (14), 255 (10), 253 (13), 237 (27), 219 (20), 209 (65), 193 (10), 175 (6), 153 (7), 137 (16) ; HRMS: ber. (MH⁺) 353.2328, gef. 353.2324.

### B3)

### (4Z,8E)-2-Acetoxy-2-acetyl-5,9-dimehtyl-10-hydroxy-deca-4,8-dien-säure-tert-butylester

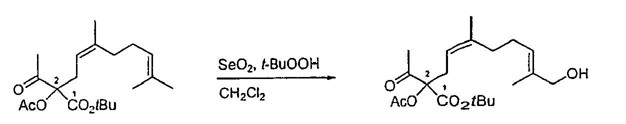

Selendioxidpulver (0.16 g, 1.42 mmol) wird in CH₂Cl₂ (50 mL) suspendiert und eine 70% *tert*-butylhydroperoxid Lösung (10.2 g, 79.5 mmol) zugefügt und die entstandene Mischung 30 min bei rt gerührt. Danach wird B2 (10.0 g, 28.4 mmol) zugegeben und weiter 48 h bei rt gerührt. Nach dem Einengen des Reaktionsgemisches im Vakuum wird Toluol (50 mL) zugefügt und wiederholt eingeengt (Entfernen von *tert*-butylhydroperoxid). Der Vorgang wird 3 mal wiederholt und das erhaltene Öl durch Flashchromatographie (PE : EE 2:1) gereingt.
¹H-NMR (250 MHz, CDCl₃): δ = 1.45 (s, 9H, C(CH₃)₃), 1.66 (s, 3H, =C(CH₃)), 1.71 (s, 3H, =C(CH₃)), 1.90-2.15 (m, 4H, CH₂-6, CH₂-7), 2.15 (s, 3H, CH₃COO), 2.31 (s, 3H, CH₃CO), 2.85-2.88 (m, 2H, CH₂-3), 3.99 (s, 2H, CH₂-10), 5.02 (m, 1H, CH-4), 5.38 (m, 1H, CH-8) ppm. C₂₀H₃₂O₆ (368.46, 368.22), MS (CI): m/z (%) = 369 (6) [M+H]⁺, 329 (6), 311 (26), 295 (100), 271 (11), 253 (24), 235 (14), 203 (10), 169 (9), 135 (10); HRMS: ber. (MH⁺) 369.2277, gef. 369.2288.

### B4)

### Z-(9S)-2-Acetoxy-2-acetyl-5,9-dimethyl-10-hydroxy-deca-4-en-säure-tert-butylester

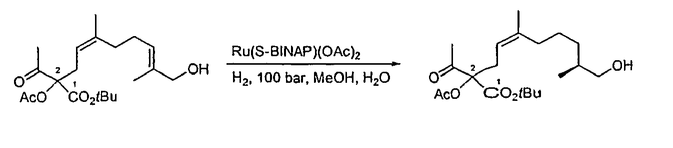

B3 (7.94 g, 21.6 mmol) wird in einer Mischung aus absolutem Methanol (15.0 mL) und Wasser (750 µl) gelöst. Die Mischung wird entgast und Ru(*S*-BINAP) (OAc)₂ (185 mg, 1 mol%) zugefügt. Die Reaktionsmischung wird in einen Autoklaven mit Rührer überführt, 5 mal mit Wasserstoff gespült und die Apparatur für 22 h unter Rühren unter einen Wasserstoffdruck von 100 bar gesetzt. Der Überdruck wird entfernt und das Gemisch im Vakuum eingeengt. Das erhaltene braune Öl wird mittels Flashchromatographie (PE : EE 2:1) gereinigt.
¹H-NMR (250 MHz, CDCl₃): δ = 0.91 (d, 3H, J = 6.4 Hz, 9-CH₃), 1.45 (s, 9H, C(CH₃)₃), 1.68 (s, 3H, 5-CH₃), 1.0 - 2.06 (m, 7H, CH₂-6,7,8, CH-9), 2.15 (s, 3H, CH₃COO), 2.31 (s, 3H, CH₃CO), 2.84 - 2.88 (m, 2H, CH₂-3), 3.41 (dd, AB, *J*₁= 10.5 Hz, *J*₂= 6.3 Hz, 1H, CH₂-10), 3.49 (dd, AB, *J*₁= 10.5 Hz, *J*₂= 5.9 Hz, 1H, CH₂-10), 5.00 (t, 1H, CH-4) ppm. C₂₀H₃₄O₆ (370.48, 370.24), MS (CI) : m/z (%) = 369 (6) [M+H]⁺; HRMS: ber. (MH⁺) 371.2433, gef. 371.2420

### B5)

### Z-(10S)-3-Acetoxy-11-hydroxy-6,10-dimethyl-5-undecen-2-on

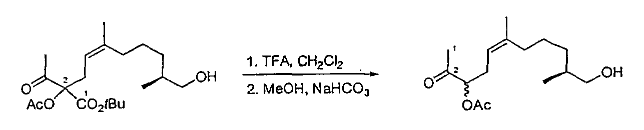

B4 (1.03 g, 2.79 mmol) wird in CH₂Cl₂ (28 mL) gelöst und TFA (2.80 mL) zugefügt. Nach 2-stündigem Rühren wird die Mischung im Vakuum eingeengt und das erhaltene Öl in Methanol (28 mL) aufgenommen. Es wird NaHCO₃ (5.6 mL) zugefügt und die Suspension für 140 min bei RT gerührt. Verdünnen mit Et₂O (200 mL), Waschen mit Wasser (2 x 50 mL), brine (50 mL), Trocknen über Na₂SO₄. und Einengen im Vakuum ergeben das Rohprodukt. Es wird durch Säulenchromatographie (PE : EE 3:2) gereinigt.
¹H-NMR (400 MHz, CDCl₃) : δ = 0.92 (d, 3H, *J*= 6.6 Hz, 10-CH₃), 1.00 - 1.20 (m, 1H), 1.30 -1.50 (m, 3H), 1.60 (m, 1H), 1.70 (s, 3H, CH₃-6), 2.01 (m, 2H), 2.14, 2.16 (2 x s, 6H, CH₃-1, CH₃COO) 2.48 (m, 2H, CH₂-4), 3.46 (m, 2H, CH₂ - 11), 4.98 (m, 1H, CH₃-3), 5.11 (m, 1H, CH₃-5) ppm. C₁₅H₂₆O₄ (270.36, 270.18), MS (ESI-MS): m/z (%) = 563.3 (100) [2M+Na]⁺, 293.0 (54) [M+Na]⁺, 271.1 (7) [M+H]⁺

### B6)

### Z-(10S)-3-Acetoxy-11-tert-butyldimethylsilyloxy-6,10-dimethyl-5-undecen-2-on

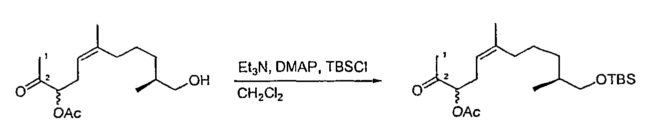

B5 (528 mg, 1.95 mmol) wird in absolutem CH₂Cl₂ (10.0 mL) gelöst. Nach erfolgter Zugabe von Triethylamin (541 µl, 3.90 mmol) und DMAP (12 mg, 0.10 mmol) wird die Reaktionsmischung auf 0°C gekühlt und TBDMSCl (368 mg, 2.44 mmol) zugefügt. Es wird über Nacht gerührt und Methanol (460 µl) zugegeben und für weitere 30 min gerührt. Nach dem Entfernen des Lösungsmittels im Vakuum wird der Rückstand durch Flashchromatographie (PE : EE 10:1) gereinigt.
¹H-NMR (400 MHz, CDCl₃): δ = 0.02 (s, 6H, Si(CH₃)₂), 0.83 (d, 3H, J= 6.4 Hz, 10-CH₃), 0.86 (s, 9H, SiC(CH₃)₃), 0.94-1.07 (m, 1H), 1.20-1.42 (m, 3H), 1.46-1.58 (m, 1H), 1.66 (s, 3H, 6-CH₃), 1.89-2.01 (m, 2H), 2.10, 2.12 (2 x s, 6H, CH₃-1, CH₃COO), 2.38-2.47 (m, 2H, CH₂-4), 3.33 (dd, 1H, J= 9.8 Hz, J= 6.4 Hz, CH₂-11), 3.39 (dd, 1H, J= 10 Hz, J= 6.0 Hz, CH₂-11), 4.94 (t, 1H, *J*=6.4 Hz, CH-3), 5.03 - 5.07 (m, 1H, CH-5) ppm. C₂₁H₄₀O₄Si (384.63, 384.27), MS (CI): m/z (%) = 385 (13) [M+H]⁺, 327 (13), 267 (26), 253 (6), 193 (40), 175 (62), 117 (100). HRMS: ber. (MH⁺) 385.2774, gef. 385.2785

### B7)

### Z-(10S)-11-(tert-Butyldimethylsilyloxy)-3-hydroxy-6,10-dimethyl-5-undecen-2-on

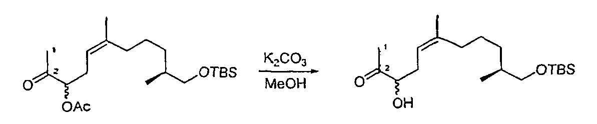

B6 (1.94 g, 5.05 mmol) wird in Methanol (20.0 ml) gelöst und eine gesättigte K₂CO₃-Lösung (400 µl) zugefügt. Nach 10 minütigem Rühren bei RT wird brine (30 mL) zugegeben und mit Et₂O (5 x 30 mL) extrahiert. Die vereinigten organischen Extrakte werden mit brine (50 mL) gewaschen, über Na₂SO₄ getrocknet und im Vakuum eingeengt. Das erhaltene Rohprodukt wird mittels Flashchromatographie (PE : EE 4:1) gereinigt.
¹H-NMR (400 MHz, CDCl₃): δ = 0.04 (s, 6H, Si(CH₃)₂), 0.86 (d, 3H, *J*=6.4 Hz, 10-CH₃), 0.89 (s, 9H, SiC(CH₃)₃), 1.00 - 1.65 (m, 5H, CH₂-8, 9, CH-10), 1.70 (s, 3H, 6-CH₃), 1.96 - 2.04 (m, 2H, CH₂-7), 2.19 (s, 3H, CH₃-1), 2.34 - 2.41, 2.52 - 2.58 (2 x m, 2H, CH₂-4), 3.30 - 3.44 (m, 2H, CH₂-11), 4.19 - 4.23 (m, 1H, CH-3), 5.08 - 5.11 (m, 1H, CH-5) ppm. C₁₉H₃₈O₃Si (342.59, 342.26), HRMS: ber. 342.2590, gef. 342.2583

### B8)

### (R)-α-Methoxyphenylessigsäure-Z-(1S,8S)-1-acetyl-9-(tert-butyldimethylsilyloxy)-4,8-dimethylnon-3-enyl ester

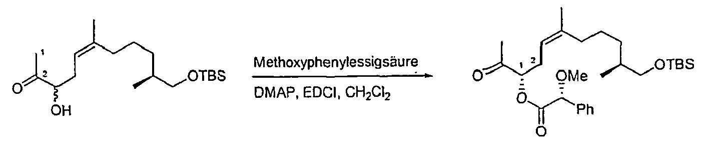

Zu einer Lösung von B7 (2.41 g, 7.04 mmol), (*R*)-α-Methoxyphenylessigsäure (1.28 g, 7.75 mmol) und DMAP (86 mg, 0.70 mmol) in CH₂Cl₂ (72.0 mL) wird EDCI (2.70 g, 14.09 mmol) zugegeben und die erhaltene Mischung für 2 h bei RT gerührt. Es wird mit Et₂O (250 mL) verdünnt, mit Wasser (2 x 100 mL) und brine (100 mL) gewaschen und die organische Phase über Na₂SO₄ getrocknet. Einengen des Lösungsmittels im Vakuum und anschließende Säulenchromatographie (PE : EE 10:1) ergeben das Produkt.
¹H-NMR (400 MHz, CDCl₃): δ = 0.01 (s, 6H, Si (CH₃)₂), 0.83 (d, 3H, *J*=6.8 Hz, 8-CH₃), 0.86 (s, 9H, SiC(CH₃)₃), 0.92 - 1.62 (m, 5H, CH₂-6, 7, CH-8), 1.63 (s, 3H, 4-CH₃), 1.76 (s, 3H, CH₃CO), 1.84 - 2.01 (m, 2H, CH₂-5), 2.34 - 2.50 (m, 2H, CH₂-2), 3.35 (dd, 1H, *J*= 9.7 Hz, *J*= 6.4 Hz, CH₂-9), 3.39 (dd, 1H, J= 9.8 Hz, *J*= 6.1 Hz, CH₂-9), 3.42 (s, 3H, OCH₃), 4.79 (s, 1H, CHOCH₃), 4.93 - 5.30 (m, 2H, CH-1, 3), 7.35-7.49 (m, 5H, phenyl-CH) ppm. C₂₈H₄₆O₅Si (490.75, 490.31), HRMS: ber. 490.3115, gef. 490.3107

### B9)

### (R)-α-Methoxyphenylessigsäure-Z-(1S,8S)-9-(tert-butyldimethylsilyloxy)-4,8-dimethyl-1-[E-1-methyl-2-(2-methylthiazol-4-yl)-vinyl]-non-3-enyl ester

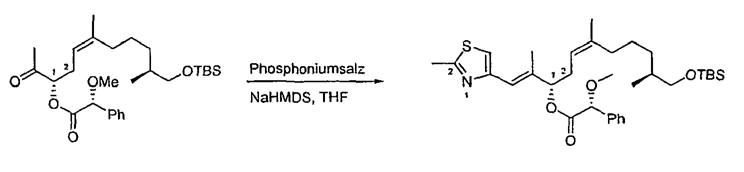

Eine Lösung von Tributyl-(2-methylthiazol-4-ylmethyl)-phosphonium chloride (1.02 g, 2.92 mmol) in abs. THF (19.0 mL) wird auf -78 °C gekühlt und es wird tropfenweise NaHMDS (2 M in THF, 1.56 mL, 3.12 mmol) zugegeben. Nach 10 minütigem Rühren, wird eine Lösung von B8 (1.19 g, 2.43 mmol) in abs. THF (8.0 mL) zugefügt und 60 min bei -78°C gerührt.

Das Reaktionsgemisch wird mit NH₄Cl (45 mL) gequencht und mit Et₂O (5 x 25 mL) extrahiert. Die vereinigten organischen Phasen werden mit Wasser (3×30 mL) und brine (1×50 mL) gewaschen über Na₂SO₄ getrocknet und das Lösungsmittel im Vakuum abgezogen. Das Rohprodukt wird mittels Flashchromatographie (PE : EE 5:1) gereingt.
¹H-NMR (300 MHz, CDCl₃): δ = 0.02 (s, 6H, Si (CH₃)₂), 0.84 (d, 3H, *J*=6.9 Hz, 8-CH₃), 0.89 (s, 9H, SiC(CH₃)₃), 0.95-2.05 (m, 7H), 1.51 (s, 3H, 4-CH₃), 2.05 (s, 3H), 2.29 (t, 2H, *J*=7.2 Hz, CH₂-2), 2.70 (s, 3H, SCCH₃), 3.30-3.46 (m, 2H, CH₂-9), 3.41 (s, 3H, OCH₃), 4.75-4.80 (m, 1H, CH-1), 4.78 (s, 1H, CHOCH₃), 5.25 (t, 1H, *J*=6.6 Hz, CH-3), 6.48 (s, 1H), 6.90 (s, 1H), 7.32-7.37 (m, 3H), 7.43-7.46 (m, 2H) ppm. C₃₃H₅₁NO₄SSi (585.91, 585.33), HRMS : ber. (MH⁺) 586.3390, gef. 586.3381

### B10)

### (1E,5Z,3S,10S)-11-(tert-Butyldimethylsilyloxy)-2,6,10-trimethyl-1-(2-methylthiazol-4-yl)-undeca-1,5-dien-3-ol

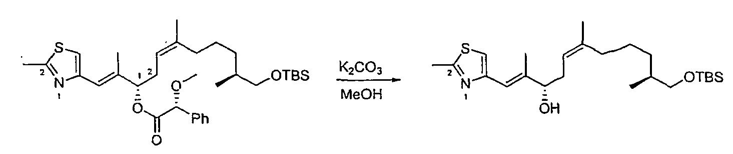

Zu einer Lösung von B9 (0.59 g, 1.00 mmol) in Methanol (10.0 mL) wird festes K₂CO₃ (0.28 g, 2.00 mmol) zugegeben und die entstandene Mischung 90 min bei RT gerührt. Das Lösungsmittel wird im Vakuum abgezogen und der Rückstand in Ethylacetat (40 mL) aufgenommen. Es wird mit Wasser (3x10 mL) und brine (10 mL) gewaschen über Na₂S0₄, getrocknet und im Vakuum eingeengt. Das Rohprodukt wird mittels Flashchromatographie (PE : EE 3:1) gereingt.
¹H-NMR (300 MHz, CDCl₃): δ = 0.03 (s, 6H, Si(CH₃)₂), 0.86 (d, *J*=6.7 Hz, 3H, 10-CH₃), 0.89 (s, 9H, SiC (CH₃)₃) 1.00-1.65 (m, 5H), 1.71 (s, 3H, 6-CH₃), 1.84 (d, 1H, OH), 2.01-2.08 (m, 2H), 2.05 (s, 3H, 2-CH₃), 2.35 (m , 2H, CH₂-4), 2.71 (s, 3H, SCCH₃), 3.35 (dd, J= 9.7 Hz, *J*=6.5 Hz, 1H, CH₂-11), 3.44 (dd, *J*=9.7 Hz, *J*=5.9 Hz, 1H, CH₂-11), 4.13 (m, 1H, CH-3), 5.16 (m, 1H, CH-5), 6.56 (s, 1H, CH-1), 6.94 (s, 1H, CHS) ppm. MS (CI): m/z (%) = 438 (13) [M+H]⁺, 420 (27), 396 (4), 380 (12), 364 (4), 259 (27), 213 (100). C₂₄H₄₃NO₂SSi (437.75, 437.28), HRMS: ber. (M+Na)⁺ 460.268, gef. 460.2676.

### B11)

### (R)-α-Methoxyphenylessigsäure-Z-(1S,8S)-9-(tert-butyldimethylsilyloxy)-4,8-dimethyl-1-[(1E)-1-methyl-2-(2-methylthiazol-4-yl)-vinyl]-non-3-enyl ester

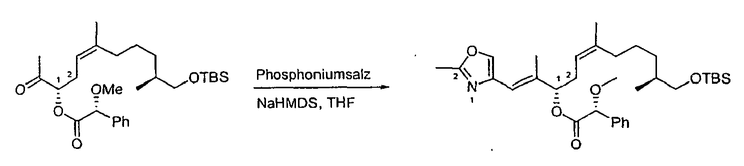

Eine Lösung von Tributyl-(2-methyloxazol-4-ylmethyl)-phosphonium bromid (0.43 g, 1.12 mmol) in abs. THF (10 mL) wird auf -78 °C gekühlt und es wird tropfenweise NaHMDS (2 M in THF, 0.64 mL, 1.12 mmol) zugegeben. Nach 10 minütigem Rühren, wird eine Lösung von B8 (0.48 g, 0.97 mmol) in abs. THF (4 mL) zugefügt und 120 min bei - 78°C gerührt. Das Reaktionsgemisch wird mit NE₄Cl (10 mL) gequencht und mit Et₂O (5 x 20 mL) extrahiert. Die vereinigten organischen Phasen werden mit Wasser (3×15 mL) und brine (1x20 mL) gewaschen über Na₂SO₄ getrocknet und das Lösungsmittel im Vakuum abgezogen. Das Rohprodukt wird mittels Flashchromatographie (PE : EE 4:1) gereingt.
¹H-NMR (300 MHz, CDCl₃): δ = 0.03 (s, 6H, Si(CH₃)₂), 0.83 (d, 3H, 8-CH₃), 0.89 (s, 9H, SiC(CH₃)₃), 0.93-1.92 (m, 7H), 1.58 (s, 3H, 4-CH₃), 1.91 (s, 3H), 2.25 (t, 2H, CH₂-2), 2.45 (s, 3H, NCCH₃), 3.32-3.39 (m, 2H, CH₂-9), 3.41 (s, 3H, OCH₃), 4.72 (t, 1H, CH-1), 4.77 (s, 1H), 5.23 (t, 1H, CH-3), 6.23 (s, 1H), 7.26-7.46 (m, 5H), 7.44 (s, 1H) ppm.

### B12)

### (1E,5Z,3S,10S)-11-(tert-Butyldimethylsilyloxy)-2,6,10-trimethyl-1-(2-methyloxazol-4-yl)-undeca-1,5-dien-3-ol

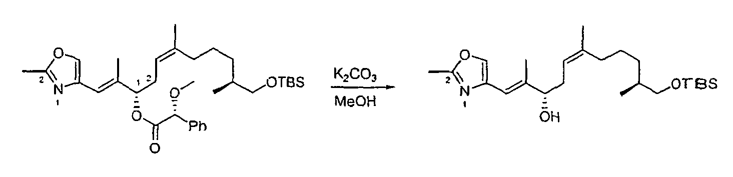

Zu einer Lösung von B9 (0.2 g, 0.35 mmol) in Methanol (10.0 mL) wird festes K₂CO₃ (95 mg) zugegeben und die entstandene Mischung 2.5 h bei RT gerührt. Das Lösungsmittel wird im Vakuum abgezogen und der Rückstand in Ethylacetat (40 mL) aufgenommen. Es wird mit Wasser (3×10 mL) und brine (10 mL) gewaschen über Na₂SO₄, getrocknet und im Vakuum eingeengt. Das Rohprodukt wird mittels Flashchromatographie (PE : EE 3:1) gereingt.
¹H-NMR (300 MHz, CDCl₃): δ = 0.01 (s, 6H, Si(CH₃)₂), 0.84 (d, 3H, 10-CH₃), 0.86 (s, 9H, SiC(CH₃)₃), 1.29-1.70 (m, 5H), 1.67 (s, 3H, 6-CH₃), 1.89 (s, 3H, 2-CH₃), 1.97-2.01 (m, 2H, CH₂-7), 2.27-2.31 (m, 2H, CH₂-4), 2.42 (s, 3H, NCCH₃), 3.32 (dd, 1H, CH₂-11), 3.40 (dd, 1H, CH₂-11), 4.05-4.13 (m, 1H, CH-3), 5.10 (t, 1H, CH-5), 6.26 (s, 1H, CH-1), 7.44 (s, 1H, OCH) ppm.
C₂₄H₄₃NC₃Si (421.69, 421.30), HRMS: ber. (M+Na)⁺ 444.2904, gef. 444.2904

### B13)

### (R)-α-Methoxyphenylessigsäure-(3Z,8E,1S)-9-(tert-butyldimethylsilyloxy)-4,8-dime-thyl -1-[(1E)-1-methyl-2-(6-methyl-pyridin-2-yl)-vinyl]-non-3,7-dienyl ester

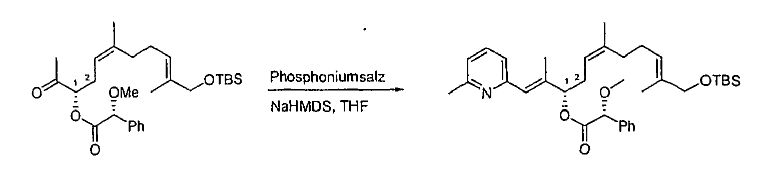

Eine Lösung von Tributyl-(6-methylpyridin-2-ylmethyl)-phosphonium chlorid (1.3 g, 3.78 mmol) in abs. THF (20 mL) wird auf -78 °C gekühlt und es wird tropfenweise NaHMDS (2 M in THF, 0.75, 2.04 ml, 4.1 mmol) zugegeben. Nach 10 minütigem Rühren, wird eine Lösung von B8 (1.59 g, 3.15 mmol) in abs. THF (5 mL) zugefügt und 3 h bei - 78°C gerührt. Das Reaktionsgemisch wird mit NH₄Cl (50 mL) gequencht und mit Et₂O (5 x 30 mL) extrahiert. Die vereinigten organischen Phasen werden mit Wasser (3x20 mL) und brine (1x50 mL) gewaschen über Na₂SO₄ getrocknet und das Lösungsmittel im Vakuum abgezogen. Das Rohprodukt wird mittels Flashchromatographie (PE : EE 8:1) gereinigt.
¹H-NMR (300 MHz, CDCl₃): δ = 0.03 (s, 6H, Si(CH₃)₂), 0.83 (d, 3H, 8-CH₃), 0.89 (s, 9H, SiC(CH₃)₃), 0.93-1.92 (m, 7H), 1.58 (s, 3H, 4-CH₃), 1.91 (s, 3H), 2.25 (t, 2H, CH₂-2), 2.45 (s, 3H, NCCH₃), 3.32-3.39 (m, 2H, CH₂-9), 3.41 (s, 3H, OCH₃), 4.72 (t, 1H, CH-1), 4.77 (s, 1H), 5.23 (t, 1H, CH-3), 6.23 (s, 1H), 6.93 (d, 1H, CH_{Pyr}), 7.06 (d, 1H, CH_{Pyr}), 7.22-7.58 (m, 6H), ppm.

### B14)

### (1E,5Z,9E,3S)-11-(tert-Butyldimethylsilyloxy)-2,6,10-trimethyl-1-(6-methyl-pyridin-2-yl-)-undeca-1,5,9-trien-3-ol

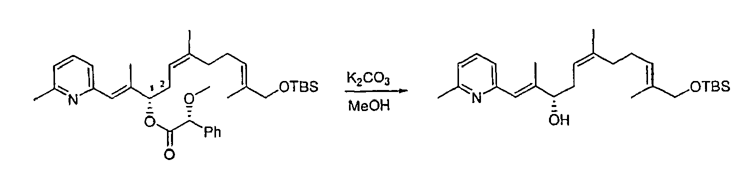

Zu einer Lösung von B9 (0.55 g, 0.96 mol) in Methanol (12 mL) wird festes K₂CO₃ (0.26 g, 1.91 mmol) zugegeben und die entstandene Mischung 2.5 h bei RT gerührt. Das Lösungsmittel wird im Vakuum abgezogen und der Rückstand in Ethylacetat (40 mL) aufgenommen. Es wird mit Wasser (3x10 mL) und brine (10 mL) gewaschen über Na₂SO₄, getrocknet und im Vakuum eingeengt. Das Rohprodukt wird mittels Flashchromatographie (PE : EE 2.5:1) gereinigt.
¹H-NMR (300 MHz, CDCl₃): = δ = 0.06 (s, 6H, Si(CH₃)₂), 0.90 (s, 9H, SiC(CH₃)₃), 1.46-2.18 (m, 4H), 1.61 (s, 3H, 10-CH₃), 1.75 (s, 3H, 6-CH₃ ), 2.05 (s, 3H, 2-CH₃), 2.36-2.40 (m, 2H, CH₂-4), 2.56 (s, 3H, NCCH₃), 4.01 (s, 2H, CH₂-11), 4.17 (t, 1H, CH-3), 5.22 (t, 1H, CH-5), 5.40 (t, 1H, CH-9), 6.59 (s, '1H, CH-1), 6.97 (d, 1H, CH-5_{Pyr}), 7.07 (d, 1H, CH-3_{Pyr}), 7.54 (d, 1H, CH-4_{Pyr})
C₂₆H₄₃NO₂Si (429.31, 429.71), HRMS: ber, (M+H)⁺ 430.3163 gef. 430.3130

### C (kombinatorische Verknüpfungen von A-B)

### C1)

### (3-Bromo-1,1-dimethyl-2-oxo-butylsulfanyl)-essigsäure-(3Z,1S,8S)-9-(tert-butyl-dimethyl-silyloxy)-4,8-dimethyl-1-[E-1-methyl-2-(2-methylthiazol-4-yl)-vinyl]-non-3-enylester

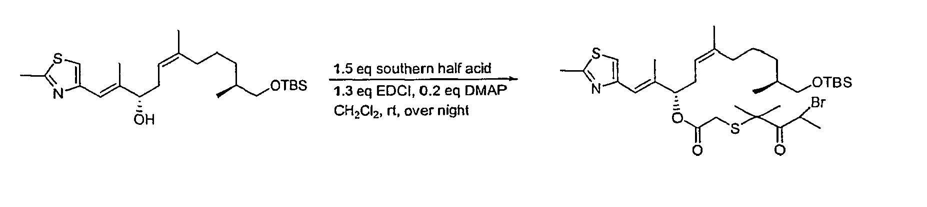

(3-Bromo-1,1-dimethyl-2-oxo-butylsulfanyl)essigsäure (A5) (0.1 g, 0.37 mmol), (1*E*,5*Z*,3*S*, 10*S*)-11-(*tert-*Butyldimethylsilyloxy)-2,6,10-trimethyl-1-(2-methylthiazol-4-yl)-undeca-1,5-dien-3-ol (B10) (0.11 g, 0.25 mmol) und DMAP (6 mg, 0.049 mmol) werden in CH₂Cl₂ (4 mL) gelöst und die Lösung auf 0°C gekühlt. Es wird EDCI (0.06 g, 0.32 mmol) zugefügt und nach 10 minütigem Rühren wird das Eisbad entfernt und die Mischung über Nacht bei RT weiter gerührt. Verdünnen mit Et₂O (100 mL), waschen mit halbkonzentrierter NaCl-Lösung (40 mL) and brine (40 mL), Trocknen über Na₂SO₄, und Entfernen des Lösungsmittels im Vakuum ergeben das Rohprodukt. Es wird mittels Flashchromatographie (PE : EE 5:1) gereinigt.
¹H-NMR (400 MHz, CDCl₃): δ = -0.08 (s, 6H, Si(CH₃)₂), 0.83 (d, *J*=6.4 Hz, 3H), 0.86 (s, 9H, SiC(CH₃)₃), 0.94 - 1.58 (m, 5H), 1.44 (s, 3H, C(CH₃)₂), 1.60 (s, 3H, C(CH₃)₂), 1.63 (s, 3H), 1.74^{*} (2 x d, 3H, *J*=6.6 Hz, CHBrCH₃), 1.96 (t, 2H), 2.03 (s, 3H), 2.29 - 2.47 (m, 2H), 2.67 (s, 3H, SCCH₃), 2.99 (d, 1H, *J*=15.4 Hz, SCH₂), 3.11, 3.12^{*} (2 x d, 1H, *J*=15.4 Hz, SCH₂), 3.32 (d, 1H, *J*=6.6 Hz, *J*=9.7 Hz CH₂O), 3.40 (d, 1H, *J*=6.6 Hz, *J*=9.7 Hz CH₂O), 5.00 (t, 1H), 5.06 (2 x q, 1H, CHBr), 5.13 - 5.24 (m, 1H, OCH), 6.46 (s, 1H), 6.92, 6.93^{*} (2 x s, 1H, CHS) ppm. (^{*}zweites Diastereomer)
C₃₂H₅₄BrNO₄S₂Si (688.89, 687.24), MS (CI): m/z (%) = 802 (0.9) [M+H]⁺, 800 (0.6) [M+H]⁺, 420 (75), 168 (100). HRMS: ber. (M+Na) 824.3557, gef. 824.3568

### C2)

### (3-Bromo-1,1-dimethyl-2-oxo-butylsulfanyl)-essigsäure-(3Z,1S,8S)-9-hydroxy-4,8-dimethyl-1-[E-1-methyl-2-(2-methyl-thiazol-4-yl)-vinyl]-non-3-enyl ester

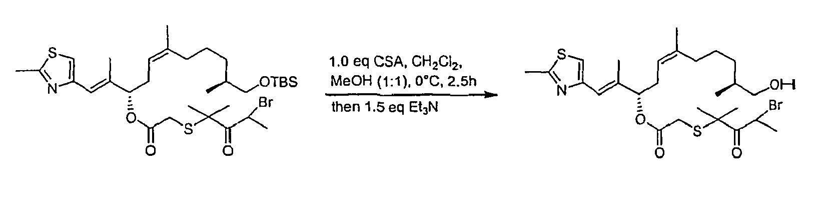

(137 mg, 0.19 mmol) wird in einer 1:1 Mischung aus CH₂Cl₂ und MeOH (5 mL) gelöst und bei 0°C wird CSA (46 mg, 0.19 mmol) zugefügt. Die Reaktionsmischung wird 2.5 h bei 0°C gerührt und anschließend wird Triethylamin (42µl, 0.29 mmol) zugegeben. Die Lösungsmittel werden im Vakuum abgezogen und das erhaltene Öl durch Flashchromatographie (PE : EE 2:1) gereinigt.
¹H-NMR (400 MHz, CDCl₃): δ = 0.90 (d, *J*=6.4 Hz, 3H, CHCH₃), 0.83 - 1.61 (m, 5H), 1.46 (s, 3H, C(CH₃)₂), 1.62 (s, 3H, C(CH₃)₂), 1.66 (s, 3H), 1.76^{*} (2 x d, 3H, *J*=6.0 Hz, CHBrCH₃), 2.00 (t, 2H, *J*=6.0 Hz), 2.05 (s, 3H), 2.09 - 2.24 (bs, 1H, OH), 2.30 - 2.54 (m, 2H), 2.69 (s, 3H, SCCH₃), 3.02 (d, 1H, *J*=14.8 Hz, SCH₂), 3.12, 3.16^{*} (2 x d, 1H, *J*=6.0 Hz, SCH₂),
3.41 (d, 1H, *J*=6.2 Hz, *J*=10.6 Hz CH₂O), 3.47 (d, 1H, *J*=6.2 Hz, *J*=10.6 Hz CH₂O), 5.04 (t, 1H), 5.08^{*} (2 x q, 1H, CHBr), 5.17 - 5.25 (m, 1H, OCH), 6.46 (s, 1H), 6.92, 6.93^{*} (2 x s, 1H, CHS) ppm. (^{*}zweites Diastereomer) HRMS: ber. (M+Na) 596.1478, gef. 596.1474

### C3)

### (3-Bromo-1,1-dimethyl-2-oxo-butylsulfanyl)-essigsäure-(3Z,1S,8S)-4,8-dimethyl-1-[E-1-methyl-2-(2-methylthiazol-4-yl)-vinyl]-9-oxo-non-3-enylester

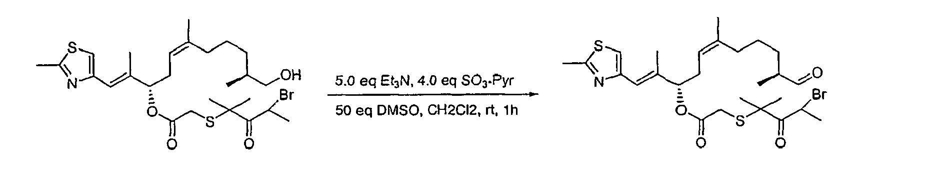

C2 (97 mg, 0.17 mmol) wird mit Triethylamin (85 mg, 0.84 mmol) und DMSO (0.66 g, 0.6 ml, 8.43 mmol) in CH₂Cl₂ (5 ml) gelöst. Bei 0°C wird SO₃-Pyridin-Komplex (107 mg, 0.67 mmol) zugegeben und die Mischung unter Argon 20 min bei 0°C und weitere 30 min bei RT gerührt. Es wird mit Et₂O (100 ml) verdünnt, mit H₂O (2 x 20 ml) und brine (2 x 20 ml) gewaschen, über Na₂SO₄ getrocknet und im Vakuum eingeengt. Das Rohprodukt wird mittels Flashchromatographie (PE : EE 4:1) gereinigt.
¹H-NMR (400 MHz, CDCl): δ = 1.02 (d, 3H), 1.20-1.65 (m, 7H), 1.40 (s, 3H, CH₃), 1.56 (s, 3H, CH₃), 1.59 (s, 3H, CH₃), 1.93-1.98 (m, 2H), 2.00 (s, 3H, CH₃), 2.22-2.46 (m, 2H), 2.63 (s, 3H, SCCH₃), 2.94-3.11 (m, 2H, SCH₂), 4.99-5.03 (m, 2H), 5.13-5.17 (m, 1H), 6.42 (s, 1H), 6.90 (s, 1H, CHS), 9.54 (s, 1H) ppm. HRMS: ber. (M+Na) 594.1318, gef. 594.1329

### C4)

### (7R,8R,9S,13Z)-8-Hydroxy-5,5,7,9,13-pentamethyl-16-[(1E)-1-methyl-2-(2-methyl-thiazol-4-yl)-vinyl]-1-oxa-4-thiacyclohexadec-13-en-2,6-dion

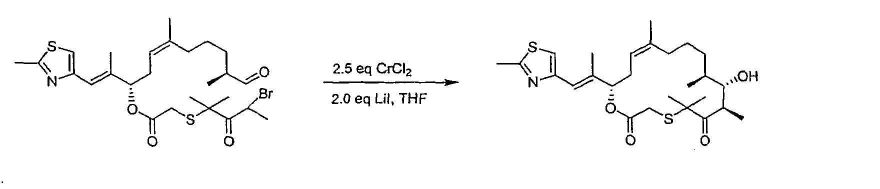

Eine Lösung von C3 (97 mg, 0.17 mmol) in THF (10 ml) wird mittels Spritzenpumpe zu einer Suspension von CrCl₂ (52 mg, 0.42 mmol) und LiI (45 mg, 0.34 mmol) in THF (15 ml) über einen Zeitraum von 80 min zugetropft. Nach erfolgter Zugabe wird noch weiter 30 min nachgerührt und anschließend mit NH₄Cl (10 ml) gequencht. Es wird mit Et₂O (3 x 20 ml) extrahiert, mit Wasser (20 ml) und brine (20 ml) gewaschen und über Na₂SO₄ getrocknet. Das Lösungsmittel wird im Vakuum abgezogen und das erhaltene Öl durch Flash-chromatographie (PE : EE 2:1) gereinigt.
¹H-NMR (500 MHz, CDCl₃) : δ = 1.01 (d, 3H, 9-CH₃ ), 1.17 (d, 3H, 7-CH₃), 1.25-1.81 (m, 7H), 1.42, 1.69 (2 x s, 6H, 5-(CH₃)₂), 2.11 (s, 3H, 1'-CH₃), 2.04-2.19 (m, 2H, 15-CH₂), 2.31-2.37 (m, 1H, CH-9), 2.70 (s, 3H, NCCH₃), 3.11, 3.25 (2 x d, 2H, CH₂-3), 3.41-3.46 (m, 1H, CH-7), 3.77-3.79 (m, 1H, CH-8), 5.12 (dd, 1H, CH-14), 5.18 (dd, 1H, CH-16), 6.54 (s, 1H), 7.26 (s, 1H, CHS) ppm.
C₂₆H₃₉NO₄S₂ (493.72, 493.23), HRMS: ber. (M+Na)⁺ 516.2212, gef. 516.2212

### C5)

### (7R,8R,9S,13Z)-8-Hydroxy-5,5,7,9,13-pentamethyl-16-[(1E)-1-methyl-2-(2-methyl-thiazol-4-yl)-vinyl]-4,4-dioxo-1-oxa-4-thia-cyclohexadec-13-en-2,6-dion

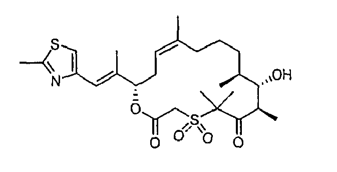

C5 wurde auf dem selben Weg wie C4 (Schritte C1 - C3 und Makrozyklisierung C4) hergestellt, jedoch ausgehend vom Südhälftenbaustein A6.
¹H-NMR (500 MHz, CDCl₃): δ = 0.99 (d, 3H, 9-CH₃ ), 1.21 (d, 3H, 7-CH₃), 1.27-1.88 (m, 7H), 1.57, 1.59 (2 x s, 6H, 5-(CH₃)₂), 1.83 (s, 3H, 13-CH₃), 2.10-2.41 (m, 3H, CH-15, CH-9), 2.13 (s, 3H, 1'-CH₃), 2.77 (s, 3H, NCCH₃), 3.56-3.58 (m, 1H, CH-7), 3.73-3.76 (m, 1H, CH-8), 3.88-3.98 (2 x d, 2H, CH₂-3), 5.00-5.06 (m, 1H, CH-14), 5.12-5.18 (m, 1H, CH-16), 6.62 (s, 1H), 7.02-7.05 (s, 1H, CHS) ppm.
C₂₆H₃₉NO₆S₂ (525.72, 525.22), HRMS: ber. (M+Na)⁺ 548.2108, gef. 548.2108

### C6)

### (7R,8R,9E,13Z)-8-Hydroxy-5,5,7,9,13-pentamethyl-16-[(1E)-1-methyl-2-(6-methyl-pyridin-2-yl)-vinyl]-1-oxa-4-thia-cyclohexadec-9,13-dien-2,6-dion

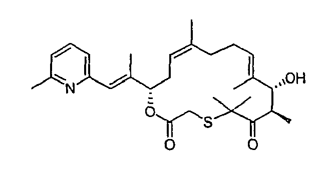

C6 wurde auf dem selben Weg wie C4 (Schritte C1 - C3 und Makrozyklisierung C4) hergestellt, jedoch ausgehend vom Nordhälftenbaustein B14.
C₂₈H₃₉NO₄S (485.68, 485.26), HRMS: ber. (M+H)⁺ 486.2671, gef. 486.26668

### C7)

### Oxidation von 3-Thia-Epothilon D (3-Thiaepothilon B oxide mix, u.a. 3-Thiaepothilon B, -sulfoxid, -sulfon)

0,5 mg 3-Thiaepothilon D wurde mit einem Überschuß einer Dimethyldioxiran-Lösung (ca. 1% in Aceton) behandelt bis alles Edukt umgesetzt war. Nach MS-Messung wird das Lösemittel im Vakuum abgezogen.
C₂₆H₃₉NO₅S₂ (509.7), MS: ber. (M+H)⁺ m/z = 510, gef. 510

### D)

### Biologische Daten (Beispiele)

Proliferationsassay GI50: Saure Phosphatase, Inkubationszeit 5 Tage, IC-50 (µM)
(Anal. Biochem. 241 (1996) 103)

| Derivat | Batch/Kommentar | MCF7 (Brustkrebs) | L 929 Mausfibroblasten | A 549 Lungenkrebs |
|---|---|---|---|---|
| MC54690 (C4) | 3-Thia-epothilon | 0,045 | 0,274 | 0,009 |
| MC54774 (C7) | 3-Thia-epo D (oxidiert) | 0,073 | 0,051 | 0,008 |
| MC54849 | D5-Typ ... | 0,531* | - | - |
| MC54847 | ... 6,7-Diastereomer | > 10* | - | - |
| MC54848 | ... 6,7-Diastereomer | > 10* | - | - |
| MC-C5 | 3-Sulfon-Epo D | > 10 | | |
| Taxol | Referenz | 0,003 | 0,270 | 0,006 |

| | | | | |
|---|---|---|---|---|
| * Inkubation 4 Tage. | | | | |

## Patentansprüche

1. Verbindung der allgemeinen Formel (I): 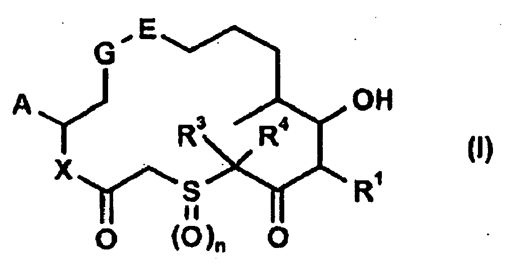 worin
A ein Heteroalkyl-, ein Heterocycloalkyl-, ein Heteroalkylcycloalkyl-, ein Heteroaryl- oder ein Heteroarylalkylrest ist,
G-E aus folgenden Gruppen ausgewählt ist, 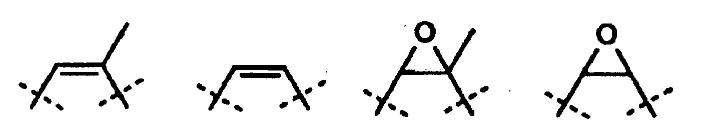
oder Teil eines gegebenenfalls substituierten Cyclopropylrings ist, wobei die Methylgruppe auch durch eine andere Alkylgruppe ersetzt sein kann,
n gleich 0, 1 oder 2 ist,
R¹ Wasserstoff, eine C₁-C₄-Alkyl- oder eine C₃-C₄-Cycloalkylgruppe ist,
X ein Sauerstoffatom oder eine Gruppe der Formel NR² ist, wobei R² ein Wasserstoffatom, OH, NH₂, NH(Alkyl), N(Alkyl)₂, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist, und
R³ und R⁴ unabhängig voneinander ein Wasserstoffatom, eine C₁-C₄-Alkylgruppe oder zusammen Teil einer Cycloalkylgruppe mit 3 oder 4 Ringatomen sind,
wobei die oben genannten Gruppen nicht substituiert oder substituiert sein können, wobei die Substituierung dergestalt ist, dass ein oder mehrere Wasserstoffatome dieser Gruppen durch F, Cl, Br, oder I, eine HO-, HS-, H₂N-, oder O₂N-Gruppe oder durch eine unsubstituierte Alkyl-, Alkenyl-, Alkinyl-, Heteroalkyl-, Cycloalkyl-, Heterocycloalkyl-, Aryl-, Heteroaryl-, Aralkyl- oder Heteroaralkylgruppen ersetzt sind;
oder ein pharmakologisch akzeptables Salz, Solvat, Hydrat oder eine pharmakologisch akzeptable Formulierung derselben.

2. Verbindung nach Anspruch 1, wobei A eine Gruppe der Formel -(CH₃)=CHR⁵, -C(C₂H₅)=CHR⁵, -C(Cl)=CHR⁵ oder -CH=CHR⁵ ist, wobei R⁵ ein Heteroaryl- oder ein Heteroarylalkylrest ist.

3. Verbindung nach Anspruch 1, wobei A die allgemeine Formel (II) bis (V), bevorzugt (II) oder (III), aufweist. 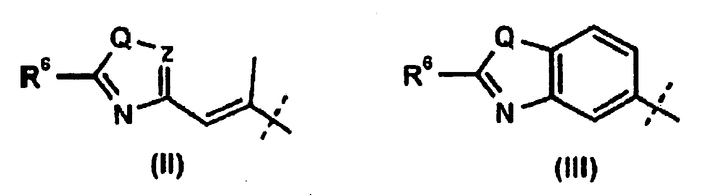 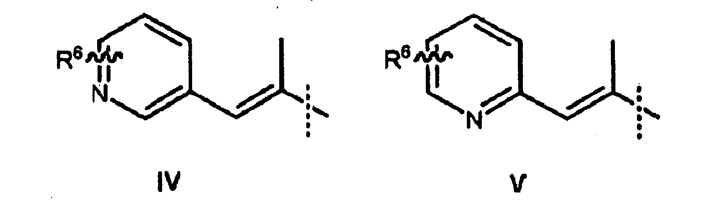 worin
Q ein Schwefelatom, ein Sauerstoffatom oder eine Gruppe der Formel NR⁷ ist, wobei
R⁷ ein Wasserstoffatom, eine C₁-C₄-Alkylgruppe oder eine C₁-C₄-Heteroalkylgruppe ist, z ein Stickstoffatom oder eine CH-Gruppe ist, und R⁶ eine Gruppe der FormelOR⁸ oder NHR⁸, eine Alkyl-, Alkenyl-, Alkinyl-, oder eine Heteroalkylgruppe ist, wobei R⁸ ein Wasserstoffatom, eine C₁-C₄-Alkylgruppe oder eine C₁-C₄-Heteroalkylgruppe ist.

4. Verbindung nach Anspruch 3, wobei z eine CH-Gruppe ist.

5. Verbindung nach Anspruch 3 oder 4, wobei Q ein Schwefelatom oder eine Sauerstoffatom ist.

6. Verbindung nach einem der Ansprüche 3 bis 5, wobei R⁶ eine Gruppe der Formel -CH₃, -CH₂OH oder -CH₂NH₂ ist.

7. Verbindung nach einem der Ansprüche 1 bis 6, wobei X ein Sauerstoffatom ist.

8. Verbindung nach einem der Ansprüche 1 bis 7, wobei R¹ eine Methylgruppe ist.

9. Verbindung nach einem der Ansprüche 1 bis 8, wobei R³ und R⁴ Methylgruppen sind.

10. Verbindung, ausgewählt aus der Gruppe, die aus (1,1-Dimethyl-2-oxo-butylsulfanyl)-essigsäureethylester, (1,1-Dimethyl-2-oxo-butylsulfanyl)-essigsäure, (2-Methyl-3-oxo-pentan-2-sulfinyl)-essigsäure, (3-Brom-1,1-dimethyl-2-oxo-butylsulfanyl)-essigsäure, (4-Brom-2-methyl-3-oxo-pentan-2-sulfonyl)-essigsäure und (2-Oxo-propylsulfanyl)-essigsäureethylester besteht, als Zwischenprodukt in der Synthese einer Verbindung nach einem der Ansprüche 1 bis 9.

11. Pharmazeutische Zusammensetzung, die eine Verbindung nach einem der Ansprüche 1 bis 9 und fakultativ Trägerstoffe und/oder Adjuvantien enthalten.

12. Verbindung nach einem der Ansprüche 1 bis 9 oder pharmazeutische Zusammensetzung nach Anspruch 11 zur Behandlung von Krebserkrankungen.

## Claims

1. Compound of the general formula (I): 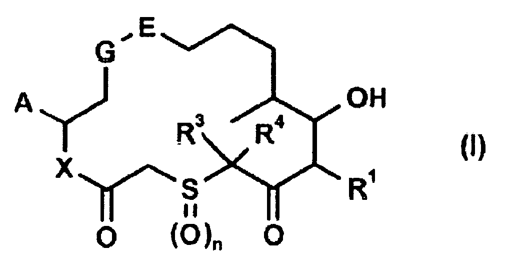 wherein
A is a heteroalkyl, a heterocycloalkyl, a heteroalkylcycloalkyl, a heteroaryl or a heteroarylalkyl group;
G-E is selected from the following groups 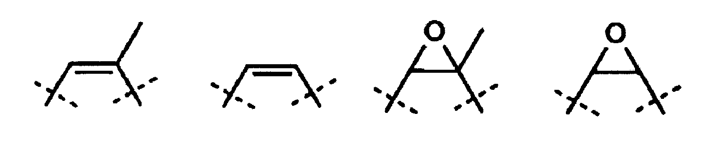
or is part of an optionally substituted cyclopropyl ring, wherein the methyl group may also be substituted by another alkyl group;
n is 0, 1 or 2;
R¹ is hydrogen, a C₁-C₄ alkyl or a C₃-C₄ cycloalkyl group;
X is oxygen or a group of the formula NR², where R² is hydrogen, OH, NH₂, NH(alkyl), N(alkyl)₂, an alkyl, an alkenyl, an alkinyl, a heteroalkyl, an aryl, a heteroaryl, a cycloalkyl, an alkylcycloalkyl, a heteroalkylcycloalkyl, a heterocycloalkyl, an aralkyl or a heteroaralkyl group; and
R³ and R⁴ are independently of each other hydrogen, a C₁-C₄ alkyl group or together part of a cycloalkyl group with 3 or 4 ring atoms;
wherein the above groups may be substituted or non-substituted, where the substitution is in such a way that one or more hydrogen atoms of these groups are replaced by F, Cl, Br or I, by a HO, HS, H₂N or O₂N group or by a non-substituted alkyl, alkenyl, alkinyl, heteroalkyl, cycloalkyl, heterocycloalkyl, aryl, heteroaryl, aralkyl or heteroaralkyl group;
or a pharmacologically acceptable salt, solvate, hydrate or a pharmacologically acceptable formulation thereof.

2. The compound according to claim 1, wherein A is a group of the formula -C(CH₃)=CHR⁵, -C(C₂H₅)=CHR⁵, -C(Cl)=CHR⁵ or -CH=CHR⁵, where R⁵ is a heteroaryl or a heteroarylalkyl group.

3. The compound according to claim 1, wherein A is a group of the general formula (II) to (V), preferably of the formula (II) or (III), 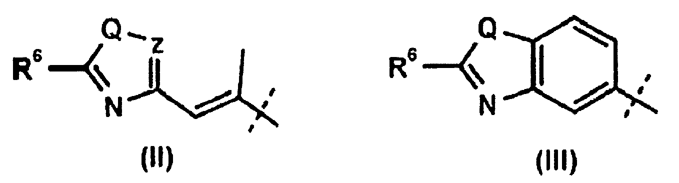 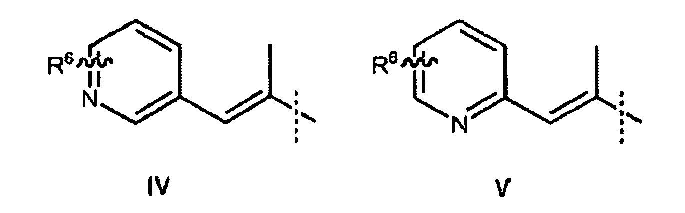 wherein
Q is sulfur, oxygen or a group of the formula NR⁷, where R⁷ is hydrogen, a C₁-C₄ alkyl group or a C₁-C₄ heteroalkyl group;
z is nitrogen or a CH group; and
R⁶ is a group of the formula OR⁸ or NHR⁸, an alkyl, an alkenyl, an alkinyl or a heteroalkyl group, where R⁸ is hydrogen, a C₁-C₄ alkyl group or a C₁-C₄ heteroalkyl group.

4. The compound according to claim 3, wherein z is a CH group.

5. The compound according to claim 3 or 4, wherein Q is sulfur or oxygen.

6. The compound according to any of claims 3 to 5, wherein R⁶ is a group of the formula -CH₃, -CH₂OH or -CH₂NH₂.

7. The compound according to any of claims 1 to 6, wherein X is oxygen.

8. The compound according to any of claims 1 to 7, wherein R¹ is a methyl group.

9. The compound according to any of claims 1 to 8, wherein R³ and R⁴ are methyl groups.

10. Compound selected from the group consisting of (1,1-dimethyl-2-oxo-butylsulfanyl)-acetic acid ethylester, (1,1-dimethyl-2-oxo-butylsulfanyl)-acetic acid, (2-methyl-3-oxo-pentan-2-sulfinyl)-acetic acid, (3-bromo-1,1-dimethyl-2-oxo-butylsulfanyl)-acetic acid, (4-bromo-2-methyl-3-oxo-pentan-2-sulfonyl)-acetic acid, and (2-oxo-propylsulfanyl)-acetic acid ethylester as an intermediate in the synthesis of a compound according to any of claims 1 to 9.

11. Pharmaceutical composition comprising a compound according to any of claims 1 to 9 and optionally carriers and/or adjuvants.

12. Compound according to any of claims 1 to 9 or pharmaceutical composition according to claim 11 for the treatment of cancer.

## Revendications

1. Composé de la formule générale (I) : 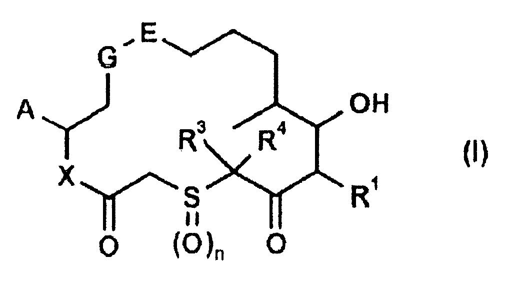 dans laquelle
A représente un radical heteroalkyle, heterocycloalkyle, heteroalkylecycloalkyle, heteroaryle ou heteroarylalkyle ;
G-E est choisi parmi des groupes suivants : 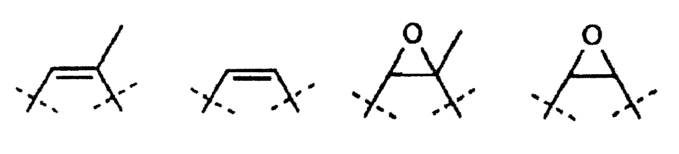
ou fait partie d'un cycle de cyclopropyle en cas échéant, dans lequel le groupe methyle peut également être substitué par un autre groupe alkyle ;
n est égale à 0, 1 ou 2 ;
R¹ représente hydrogène, un groupe C₁-C₄-alkyle ou un groupe C₃-C₄-cycloalkyle ;
X représente un atome d'oxygene ou un groupe de la formule NR₂, dans laquelle R² représente un atome d'hydrogène, OH, NH₂, NH(alkyle), N(alkyle)₂, un radical alkyle, alcényle, alcynyle, heteroalkyle, aryle, heteroaryle, cycloalkyle, alkylecycloalkyle, heteroalkylecycloalkyle, heterocycloalkyle, aralkyle ou heteroaralkyle ; et
R³ et R⁴ représentent, indépendamment l'un de l'autre, un atome d'hydrogène, un groupe C₁-C₄-alkyle ou ensemble font partie d'un groupe cycloalkyle ayant 3 ou 4 atomes de cycle,
les groupes susdits pouvant être substitués ou non, la substitution étant telle, que un ou plusieurs atomes d'hydrogène de ces groupes sont substitués par F, Cl, Br ou I, un groupe OH, HS, H₂N ou O₂N ou par un groupe alkyle, alcényle, alcynyle, heteroalkyle, cycloalkyle, heterocycloalkyle, aryle, heteroaryle, aralkyle ou heteroarylalkyle non substitué ;
ou un sel pharmacologiquement acceptable, un solvant, hydrate ou un formulation pharmacologiquement acceptable de ceux-ci.

2. Composé selon la revendication 1, dans lequel A représente un groupe de la formule -C(CH₃)=CHR⁵, C(C₂H₅)=CHR⁵ ou -CH=CHR⁵, dans laquelle R⁵ représente un radical heteroaryle ou heteroarylalkyle.

3. Composé selon la revendication 1, dans lequel A exhibite la formule genérale (II) à (V), préférentiellement (II) ou (III) 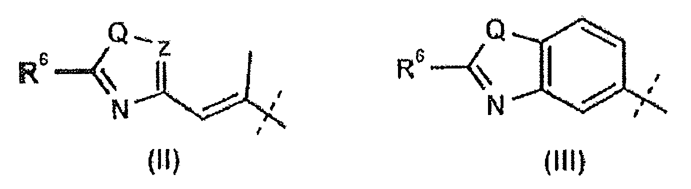 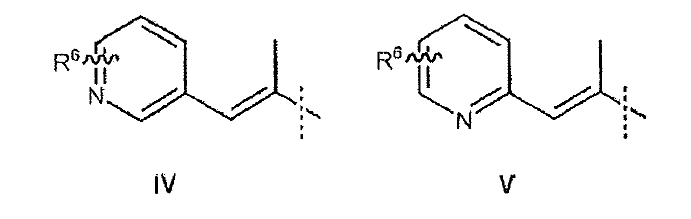 dans laquelle
Q représente un atome de soufre, un atome d'oxygène ou un groupe de la formule NR⁷, dans laquelle R⁷ représente un atome d'hydrogène, un groupe C₁-C₄-alkyle ou un groupe C₁-C₄-heteroalkyle, z représente un atome d'azote, ou un groupe CH ; et R⁶ représente un groupe de la formule OR⁸ ou NHR⁸, un groupe alkyle, alcényle, alcinyle ou heteroalkyle, dans lequel R⁸ représente un atome d'hydrogène, un groupe C₁-C₄-alkyle ou un groupe C₁-C₄-heteroalkyle.

4. Composé selon la revendication 3, dans lequel z représente un groupe CH.

5. Composé selon la revendication 3 ou 4, dans lequel Q représente un atome de soufre ou un atome d'oxygène.

6. Composé selon l'un des revendications 3 à 5, dans lequel R⁶ représente un groupe de la formule -CH₃, -CH₂OH ou -CH₂NH₂.

7. Composé selon l'un des revendications 1 à 6, dans lequel X représente un atome d'oxygène.

8. Composé selon l'un des revendications 1 à 7, dans lequel R¹ représente un groupe methyle.

9. Composé selon l'un des revendications 1 à 8, dans lequel R³ et R⁴ représentent des groupes methyle.

10. Composé choisi parmi le groupe comprenant l'ester d'éthyle de l'acide acétique de (1,1-dimethylamino-2-oxo-butylesulfanyle), l'acide acétique de (1,1-dimethylamino-2-oxo-butylesulfanyle), l'acide acétique de (3-bromo-1,1-dimethyle-2-oxo-butylesulfanyle), l'acide acétique de (4-bromo-2-methyle-2-oxo-pentane-2-sulfonyle) et l'ester d'éthyle de l'acide acétique de (2-oxo-propylesulfanyle), comme produit intermédiaire dans la synthèse d'un compose selon l'un des revendications 1 à 9.

11. Composition pharmaceutique, qui contient un composé selon l'un des revendications 1 à 9 et facultativement un excipient ou un adjuvans.

12. Composé selon l'un des revendications 1 à 9 ou une composition pharmaceutique selon la revendication 11 pour le traitement de maladies cancéreuses.
